# EUROPEAN PATENT SPECIFICATION

(11) **EP 0 782 997 B1**
(45) Date of publication and mention of the grant of the patent: **26.04.2000**
(21) Application number: 97105710.4
(22) Date of filing: 28.12.1992
(51) Int. Cl.: C07D 487/04

(54) **Triazolopyrimidine derivatives**
Triazolopyrimidinderivate
Dérivés de la triazolopyrimidine

(30) Priority: 30.12.1991 EP 91122422
(43) Date of publication of application: 09.07.1997
(62) Divisional of application: 92204097.7
(73) Proprietor: SHELL INTERNATIONALE RESEARCH MAATSCHAPPIJ B.V., 2596 HR Den Haag (NL)
(72) Inventor: Pees, Klaus-Jurgen, 55129 Mainz (DE); Albert, Guido, 55546 Hackenheim (DE)
(74) Representative: Allam, Peter Clerk

(56) References cited:
- No relevant documents disclosed

## Description

This invention relates to certain triazolopyrimidine derivatives, and a process for their preparation.

According to the invention there is therefore provided a compound of the general formula 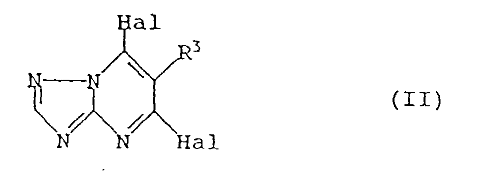 in which R³ represents an optionally substituted phenyl or naphthyl group, wherein optional substituents are selected from halogen atoms, nitro, cyano, thiocyanato, cyanato, hydroxyl, C₁₋₁₂ alkyl, C₁₋₁₂ haloalkyl, C₁₋₁₂ alkoxy, C₁₋₁₂ haloalkoxy, amino, C₁₋₁₂ alkylamino, di-C₁₋₁₂ alkylamino, formyl, C₁₋₁₂ alkoxycarbonyl, carboxyl, C₁₋₁₂ alkanoyl, C₁₋₁₂ alkylthio, C₁₋₁₂ alkylsulphinyl, C₁₋₁₂ alkylsulphonyl, carbamoyl, C₁₋₁₂ alkylamido and 3- to 6-membered heterocyclyl groups and optionally substituted phenyl, phenoxy, benzyl, benzyloxy and C₃₋₈cycloalkyl groups each optionally substituted by one or more halogen atoms, nitro, cyano, C₁₋₁₂ alkyl, C₁₋₁₂ haloalkyl, C₁₋₁₂ alkoxy or C₁₋₁₂ haloalkoxy groups; and Hal represents a chlorine or bromine atom.

It is preferred that R³ represents a phenyl or naphthyl group, each group being optionally substituted by one or more substituents selected from halogen atoms, nitro, cyano, hydroxyl, C₁₋₁₂ alkyl, C₁₋₁₂ haloalkyl, C₁₋₁₂ alkoxy, C₁₋₁₂ haloalkoxy, amino, C₁₋₄ alkylamino, di-C₁₋₄ alkylamino, formyl, C₁₋₄ alkoxycarbonyl, carboxyl, phenyl, phenoxy and benzyloxy groups.

More preferably, R³ represents a phenyl group optionally substituted by up to three substituents selected from halogen atoms, C₁₋₄ alkyl, C₁₋₄ haloalkyl, C₁₋₄ alkoxy, C₁₋₄ haloalkoxy, phenyl, phenoxy and benzyloxy groups, or a naphthyl group.

A particularly preferred sub-group of compounds of formula II is that in which R³ represents a phenyl, fluorophenyl, chlorophenyl, bromophenyl, chloro-fluorophenyl, methylphenyl, propylphenyl, trifluoromethylphenyl, methoxyphenyl, ethoxyphenyl, dimethoxyphenyl, trimethoxyphenyl, trifluoromethoxyphenyl, biphenylyl, phenoxyphenyl, benzyloxyphenyl or naphthyl group, It is especially preferred that the compound of formula II is selected from
5,7-dichloro-6-(4-methylphenyl)-1,2,4-triazolo[1,5-a]pyrimidine,
5,7-dibromo-6-phenyl-1,2,4-triazolo[1,5-a]pyrimidine,
5,7-dichloro-6-(4-methoxyphenyl)-1,2,4-triazolo[1,5-a]pyrimidine,
5,7-dichloro-6-(2-methoxyphenyl)-1,2,4-triazolo[1,5-a]pyrimidine,
5,7-dichloro-6-(3-methoxyphenyl)-1,2,4-triazolo[1,5-a]pyrimidine,
5,7-dichloro-6-(4-ethoxyphenyl)-1,2,4-triazolo[1,5-a]pyrimidine,
5,7-dichloro-6-phenyl-1,2,4-triazolo[1,5-a]pyrimidine,
5,7-dichloro-6-(3-chlorophenyl)-1,2,4-triazolo[1,5-a]pyrimidine,
5,7-dichloro-6-(3-trifluoromethylphenyl)-1,2,4-triazolo[1,5-a]-pyrimidine,
5,7-dichloro-6-(4-isopropylphenyl)-1,2,4-triazolo[1,5-a]pyrimidine,
5,7-dichloro-6-(4-trifluoromethoxyphenyl)-1,2,4-triazolo[1,5-a]-pyrimidine,
5,7-dichloro-6-naphth-2-yl-1,2,4-triazolo[1,5-a]pyrimidine,
5,7-dichloro-6-(3,4-dimethoxyphenyl)-1,2,4-triazolo[1,5-a]-pyrimidine,
5,7-dichloro-6-(2-chlorophenyl)-1,2,4-triazolo[1,5-a]pyrimidine,
5,7-dichloro-6-(4-fluorophenyl)-1,2,4-triazolo[1,5-a]pyrimidine,
5,7-dichloro-6-(4-biphenylyl)-1,2,4-triazolo[1,5-a]pyrimidine,
5,7-dichloro-6-(2-bromophenyl)-1,2,4-triazolo[1,5-a]pyrimidine,
5,7-dichloro-6-(2-fluorophenyl)-1,2,4-triazolo[1,5-a]pyrimidine,
5,7-dichloro-6-(4-phenoxyphenyl)-1,2,4-triazolo[1,5-a]pyrimidine,
5,7-dichloro-6-(4-benzyloxyphenyl)-1,2,4-triazolo[1,5-a]pyrimidine,
5,7-dichloro-6-(4-bromophenyl)-1,2,4-triazolo[1,5-a]pyrimidine,
5,7-dichloro-6-(3-bromophenyl)-1,2,4-triazolo[1,5-a]pyrimidine,
5,7-dichloro-6-(3-fluorophenyl)-1,2,4-triazolo[1,5-a]pyrimidine,
5,7-dichloro-6-(2-benzyloxyphenyl)-1,2,4-triazolo[1,5-a]pyrimidine,
5,7-dichloro-6-(2,3-dimethoxyphenyl)-1,2,4-triazolo[1,5-a]-pyrimidine,
5,7-dichloro-6-(3,4,5-trimethoxyphenyl)-1,2,4-triazolo[1,5-a]-pyrimidine, and
5,7-dichloro-6-(2-chloro-6-fluorophenyl)-1,2,4-triazolo[1,5-a]-pyrimidine,

The present invention also provides a process for the preparation of a compound of formula II as defined above which comprises reacting a compound of the general formula 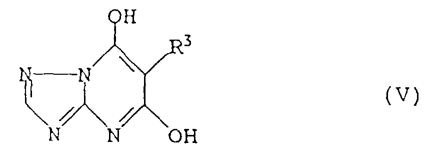 in which R³ is as defined above, with a chlorinating or brominating agent, such as phosphorus oxychloride or phosphorus oxybromide.

Compounds of formula V can be prepared by reacting 3-amino-1,2,4-triazole with an appropriate malonic acid ester under alkaline conditions according to the method of Y. Makisumi, Chem. Pharm. Bull., 9, 801, (1961).

The compounds of formula II are useful as intermediates in the preparation of certain fungicidal triazolopyrimidine derivatives which form the subject of EP-B-0 550 113 from which the present application is divided.

## Claims

1. A compound of the general formula II 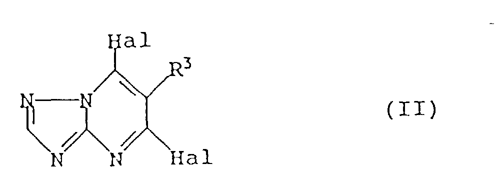 in which R³ represents an optionally substituted phenyl or naphthyl group, wherein optional substituents are selected from halogen atoms, nitro, cyano, thiocyanato, cyanato, hydroxyl, C₁₋₁₂ alkyl, C₁₋₁₂ haloalkyl, C₁₋₁₂ alkoxy, C₁₋₁₂ haloalkoxy, amino, C₁₋₁₂ alkylamino, di-C₁₋₁₂ alkylamino, formyl, C₁₋₁₂ alkoxycarbonyl, carboxyl, C₁₋₁₂ alkanoyl, C₁₋₁₂ alkylthio, C₁₋₁₂ alkylsulphinyl, C₁₋₁₂ alkylsulphonyl, carbamoyl, C₁₋₁₂ alkylamido and 3- to 6-membered heterocyclyl groups and optionally substituted phenyl, phenoxy, benzyl, benzyloxy and C₃₋₈cycloalkyl groups each optionally substituted by one or more halogen atoms, nitro, cyano, C₁₋₁₂ alkyl, C₁₋₁₂ haloalkyl, C₁₋₁₂ alkoxy or C₁₋₁₂ haloalkoxy groups; and Hal represents a chlorine or bromine atom.

2. A compound according to Claim 1 in which R³ represents a phenyl or naphthyl group, each group being optionally substituted by one or more substituents selected from halogen atoms, nitro, cyano, hydroxyl, C₁₋₁₂ alkyl, C₁₋₁₂ haloalkyl, C₁₋₁₂ alkoxy, C₁₋₁₂ haloalkoxy, amino, C₁₋₄ alkylamino, di-C₁₋₄ alkylamino, formyl, C₁₋₄ alkoxycarbonyl, carboxyl, phenyl, phenoxy, benzyl and benzyloxy groups.

3. A compound according to Claim 1 or Claim 2 in which R³ represents a phenyl group optionally substituted by up to three substituents selected from halogen atoms, C₁₋₄ alkyl, C₁₋₄ haloalkyl, C₁₋₄ alkoxy, C₁₋₄ haloalkoxy, phenyl, phenoxy and benzyloxy groups, or a naphthyl group.

4. A compound according to any one of Claims 1 to 3 in which R³ represents a phenyl, fluorophenyl, chlorophenyl, bromophenyl, chlorofluorophenyl, methylphenyl, propylphenyl, trifluoromethylphenyl, methoxyphenyl, ethoxyphenyl, dimethoxyphenyl, trimethoxyphenyl, trifluoromethoxyphenyl, biphenylyl, phenoxyphenyl, benzyloxyphenyl or naphthyl group.

5. A compound according to any one of the preceding claims selected from
5,7-dichloro-6-(4-methylphenyl)-1,2,4-triazolo[1,5-a]pyrimidine,
5,7-dibromo-6-phenyl-1,2,4-triazolo[1,5-a]pyrimidine, 5,7-dichloro-6-(4-methoxyphenyl)-1,2,4-triazolo[1,5-a]pyrimidine,
5,7-dichloro-6-(2-methoxyphenyl)-1,2,4-triazolo[1,5-a]pyrimidine,
5,7-dichloro-6-(3-methoxyphenyl)-1,2,4-triazolo[1,5-a]pyrimidine,
5,7-dichloro-6-(4-ethoxyphenyl)-1,2,4-triazolo[1,5-a]pyrimidine,
5,7-dichloro-6-phenyl-1,2,4-triazolo[1,5-a]pyrimidine, 5,7-dichloro-6-(3-chlorophenyl)-1,2,4-triazolo[1,5-a]pyrimidine,
5,7-dichloro-6-(3-trifluoromethylphenyl)-1,2,4-triazolo[1,5-a]pyrimidine,
5,7-dichloro-6-(4-isopropylphenyl)-1,2,4-triazolo[1,5-a]pyrimidine,
5,7-dichloro-6-(4-trifluoromethoxyphenyl)-1,2,4-triazolo[1,5-a]pyrimidine,
5,7-dichloro-6-naphth-2-yl-1,2,4-triazolo[1,5-a]-pyrimidine,
5,7-dichloro-6-(3,4-dimethoxyphenyl)-1,2,4-triazolo[1,5-a]pyrimidine,
5,7-dichloro-6-(2-chlorophenyl)-1,2,4-triazolo[1,5-a]pyrimidine,
5,7-dichloro-6-(4-fluorophenyl)-1,2,4-triazolo[1,5-a]pyrimidine,
5,7-dichloro-6-(4-biphenylyl)-1,2,4-triazolo[1,5-a]pyrimidine,
5,7-dichloro-6-(2-bromophenyl)-1,2,4-triazolo[1,5-a]pyrimidine,
5,7-dichloro-6-(2-fluorophenyl)-1,2,4-triazolo[1,5-a]pyrimidine,
5,7-dichloro-6-(4-phenoxyphenyl)-1,2,4-triazolo[1,5-a]pyrimidine,
5,7-dichloro-6-(4-benzyloxyphenyl)-1,2,4-triazolo[1,5-a]pyrimidine,
5,7-dichloro-6-(4-bromophenyl)-1,2,4-triazolo[1,5-a]pyrimidine,
5,7-dichloro-6-(3-bromophenyl)-1,2,4-triazolo[1,5-a]pyrimidine,
5,7-dichloro-6-(3-fluorophenyl)-1,2,4-triazolo[1,5-a]pyrimidine,
5,7-dichloro-6-(2-benzyloxyphenyl)-1,2,4-triazolo[1,5-a]pyrimidine,
5,7-dichloro-6-(2,3-dimethoxyphenyl)-1,2,4-triazolo[1,5-a]pyrimidine,
5,7-dichloro-6-(3,4,5-trimethoxyphenyl)-1,2,4-triazolo [1,5-a] pyrimidine, and
5,7-dichloro-6-(2-chloro-6-fluorophenyl)-1,2,4-triazolo[1,5-a]pyrimidine,

6. A process for the preparation of a compound of formula II as defined in any one of the preceding claims which comprises reacting a compound of the general formula V 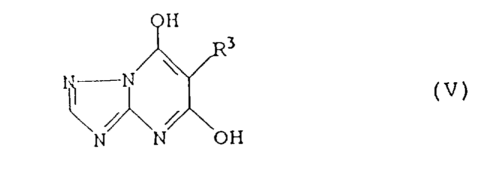 in which R³ is as defined in any one of the preceding claims, with a chlorinating or brominating agent.

7. A process according to claim 6 in which the chlorinating or brominating agent is phosphorus oxychloride or phosphorus oxybromide.

## Patentansprüche

1. Verbindung der allgemeinen Formel (II) 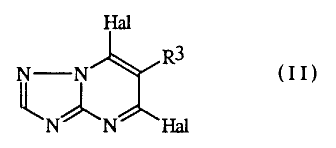 in der R³ eine gegebenenfalls substituierte Phenyl- oder Naphthylgruppe repräsentiert, worin wahlweise Substituenten ausgewählt sind aus Halogenatomen, Nitro-, Cyan-, Thiocyanat-, Cyanat-, Hydroxyl-, C₁₋₁₂-Alkyl-, C₁₋₁₂-Halogenalkyl-, C₁₋₁₂-Alkoxy-, C₁₋₁₂-Halogenalkoxy-, Amino-, C₁₋₁₂-Alkylamino-, Di-C₁₋₁₂-Alkylamino-, Formyl-, C₁₋₁₂-Alkoxycarbonyl-, Carboxyl-, C₁₋₁₂-Alkanoyl-, C₁₋₁₂-Alkylthio., C₁₋₁₂-Alkylsulfinyl., C₁₋₁₂-Alklsulfonyl-, Carbamoyl-, C₁₋₁₂-Alkylamido- und 3- bis 6-gliederigen heterocyclischen Gruppen und gegebenenfalls substituierten Phenyl-, Phenoxy-, Benzyl-, Benzyloxy- und C₃₋₈-Cycloalkylgruppen, die jeweils wahlweise substituiert sind durch ein oder mehrere Halogenatome, Nitro-, Cyan-, C₁₋₁₂-Alkyl-, C₁₋₁₂-Halogenalkyl-, C₁₋₁₂-Alkoxy- oder C₁₋₁₂-Halogenalkoxygruppen, und Hal ein Chlor- oder Bromatom repräsentiert.

2. Verbindung nach Anspruch 1, bei der R³ eine Phenyl- oder Naphthylgruppe repräsentiert, die jeweils wahlweise substituiert ist durch ein oder mehrere Substituenten, ausgewählt aus Halogenatomen, Nitro-, Cyan-, Hydroxyl-, C₁₋₁₂-Alkyl-, C₁₋₁₂-Halogenalkyl-, C₁₋₁₂-Alkoxy-, C₁₋₁₂-Halogenalkoxy-, Amino-, C₁₋₄-Alkylamino, Di-C₁₋₄-Alkylamino-, Formyl-, C₁₋₄-Alkoxycarbonyl-, Carboxyl-, Phenyl-, Phenoxy- Benzyl- und Benzyloxygruppen.

3. Verbindung nach Anspruch 1 oder 2, bei der R³ eine Phenylgruppe repräsentiert, die gegebenenfalls durch bis zu drei Substituenten substituiert ist, ausgewählt aus Halogenatomen, C₁₋₄-Alkyl-, C₁₋₄-Halogenalkyl-, C₁₋₄-Alkoxy-, C₁₋₄-Halogenalkoxy-, Phenyl-, Phenyloxy- und Benzyloxygruppen oder einer Naphthylgruppe.

4. Verbindung nach einem der Ansprüche 1 bis 3, bei der R³ eine Phenyl-, Fluorphenyl-, Chlorphenyl-, Bromphenyl-, Chlorfluorphenyl-, Methylphenyl-, Propylphenyl-, Trifluormethylphenyl-, Methoxyphenyl-, Ethoxyphenyl-, Dimethoxyphenyl-, Trimethoxyphenyl-, Trifluormethoxyphenyl-, Biphenylyl-, Phenoxyphenyl-, Benzyloxyphenyl- oder Naphthylgruppe repräsentiert.

5. Verbindung nach einem der vorhergehenden Ansprüche, ausgewählt aus 5,7-Dichlor-6-(4-methylphenyl)-1,2,4-triazolo[1,5-a]pyrimidin, 5,7-Dibrom-6-phenyl-1,2,4-triazolo[1,5-a]pyrimidin, 5,7-Dichlor-6-(4-methoxyphenyl)-1,2,4-triazolo[1,5-a]pyrimidin, 5,7-Dichlor-6-(2-methoxyphenyl)-1,2,4-triazolo[1,5-a]pyrimidin, 5,7-Dichlor-6-(3-methoxyphenyl)-1,2,4-triazolo[1,5-a]pyrimidin, 5,7-Dichlor-6-(4-ethoxyphenyl)-1,2,4-triazolo[1,5-a]pyrimidin, 5,7-Dichlor-6-phenyl-1,2,4-triazolo[1,5-a]pyrimidin, 5,7-Dichlor-6-(3-chlorphenyl)-1,2,4-triazolo[1,5-a]pyrimidin, 5,7-Dichlor-6-(4-trifluormethylphenyl)-1,2,4-triazolo[1,5-alpyrimidin, 5,7-Dichlor-6-(4-isopropylphenyl)-1,2,4-triazolo[1,5-a]pyrimidin, 5,7-Dichlor-6-(4-trifluormethoxyphenyl)-1,2,4-triazolo[1,5-a]pyrimidin, 5,7-Dichlor-6-naphth-2-yl-1,2,4-triazolo[1,5-a]pyrimidin, 5,7-Dichlor-6-(3,4-imethoxyphenyl)-1,2,4-triazolo[1,5-a]pyrimidin, 5,7-Dichlor-6-(2-chlorphenyl)-1,2,4-triazolo[1,5-a]pyrimidin, 5,7-Dichlor-6-(4-fluorphenyl)-1,2,4-triazolo[1,5-a]pyrimidin, 5,7-Dichlor-6-(4-biphenylyl)-1,2,4-triazolo[1,5.a]pyrimidin, 5,7-Dichlor-6-(2-bromphenyl)-1,2,4-triazolo[1,5-a]pyrimidin, 5,7-Dichlor-6-(2-fluorphenyl)-1,2,4-triazolo[1,5-a]pyrimidin, 5,7-Dichlor-6-(4-phenoxyphenyl)-1,2,4-triazolo[1,5-a]pyrimidin, 5,7-Dichlor-6-(4-benzyloxyphenyl)-1,2,4-triazolo[1,5-a]pyrimidin, 5,7-Dichlor-6-(4-bromphenyl)-1,2,4-triazolo[1,5-a]pyrimidin, 5,7-Dichlor-6-(3-bromphenyl)-1,2,4-triazolo[1,5-a]pyrimidin, 5,7-Dichlor-6-(3-fluorphenyl)-1,2,4-triazolo[1,5-a]pyrimidin, 5,7-Dichlor-6-(2-benzyloxyphenyl)-1,2,4-triazolo[1,5-a]pyrimidin, 5,7-Dichlor-6-(2,3-dimethoxyphenyl)-1,2,4-triazolo[1,5-a]pyrimidin, 5,7-Dichlor-6-(3,4,5-trimethoxyphenyl)-1,2,4-triazolo[1,5-a]pyrimidin und 5,7-Dichlor-6-(2-chlor-6-fluorphenyl)-1,2,4-triazolo[1,5-a]pyrimidin.

6. Verfahren zum Herstellen einer Verbindung der Formel II, wie in einem der vorhergehenden Ansprüche definiert, umfassend das Umsetzen einer Verbindung der allgemeinen Formel V 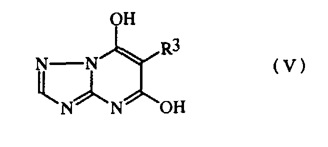 worin R³ wie in einem der vorhergehenden Ansprüche definiert ist, mit einem Chlorierungs- oder Bromierungsmittel.

7. Verfahren nach Anspruch 6, worin das Chlorierungs- oder Bromierungsmittel Phosphoroxychlorid oder Phosphoroxybromid ist.

## Revendications

1. Composé de formule générale : 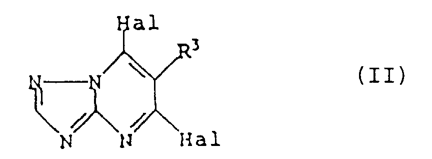 dans laquelle R³ représente un groupe phényle ou naphtyle, éventuellement substitué, les substituants facultatifs étant choisis parmi les atomes d'halogène, les groupes nitro, cyano, thiocyanato, cyanato, hydroxyle, alkyle en C₁-C₁₂, haloalkyle en C₁-C₁₂, alkoxy en C₁-C₁₂, haloalkoxy en C₁-C₁₂, amino, alkylamino en C₁-C₁₂, di-(alkyl en C₁-C₁₂)amino, formyle, (alkoxy en C₁-C₁₂)carbonyle, carboxyle, alcanoyle en C₁-C₁₂, alkylthio en C₁-C₁₂, alkylsulfinyle en C₁-C₁₂, alkylsulfonyle en C₁-C₁₂, carbamoyle, alkylamido en C₁-C₁₂ et les groupes hétérocycliques à 3 jusqu'à 6 chaînons et les groupes phényle, phénoxy, benzyle, benzyloxy et cycloalkyle en C₃-C₈ respectivement éventuellement substitués par un ou plusieurs atomes d'halogène, groupes nitro, cyano, alkyle en C₁-C₁₂, haloalkyle en C₁-C₁₂, alkoxy en C₁-C₁₂ ou haloalkoxy en C₁-C₁₂ ; et Hal représente un atome de chlore ou de brome.

2. Composé selon la revendication 1, dans lequel R³ représente un groupe phényle ou naphtyle, chaque groupe étant éventuellement substitué par un ou plusieurs substituants choisis parmi les atomes d'halogène, les groupes nitro, cyano, hydroxyle, alkyle en C₁-C₁₂, haloalkyle en C₁-C₁₂, alkoxy en C₁-C₁₂, haloalkoxy en C₁-C₁₂, amino, alkylamino en C₁-C₄, di-(alkyl en C₁-C₄)amino, formyle, (alkoxy en C₁-C₄)carbonyle, carboxyle, phényle, phénoxy, benzyle et benzyloxy.

3. Composé selon la revendication 1 ou 2, dans lequel R³ représente un groupe phényle éventuellement substitué par jusqu'à 3 trois substituants choisis parmi les atomes d'halogène, les groupes alkyle en C₁-C₄, haloalkyle C₁-C₄, alkoxy en C₁-C₄, haloalkoxy en C₁-C₄, phényle, phénoxy et benzyloxy, ou un groupe naphtyle.

4. Composé selon l'une quelconque des revendications 1 à 3, dans lequel R³ représente un groupe phényle, fluorophényle, chlorophényle, bromophényle, chlorofluorophényle, méthylphényle, propylphényle, trifluorométhylphényle, méthoxyphényle, éthoxyphényle, diméthoxyphényle, triméthoxyphényle, trifluorométhoxyphényle, biphénylyle, phénoxyphényle, benzyloxyphényle ou naphtyle.

5. Composé selon l'une quelconque des revendications précédentes, choisi parmi :
la 5,7-dichloro-6-(4-méthylphényl)-1,2,4-triazolo-[1,5-a]pyrimidine,
la 5,7-dibromo-6-phényl-1,2,4-triazolo[1,5-a]-pyrimidine,
la 5,7-dichloro-6-(4-méthoxyphényl)-1,2,4-triazolo-[1,5-a]pyrimidine,
la 5,7-dichloro-6-(2-méthoxyphényl)-1,2,4-triazolo-[1,5-a]pyrimidine,
la 5,7-dichloro-6-(3-méthoxyphényl)-1,2,4-triazolo-[1,5-a]pyrimidine,
la 5,7-dichloro-6-(4-éthoxyphényl)-1,2,4-triazolo-[1,5-a]pyrimidine,
la 5,7-dichloro-6-phényl-1,2,4-triazolo-[1,5-a]pyrimidine,
la 5,7-dichloro-6-(3-chlorophényl)-1,2,4-triazolo-[1,5-a]pyrimidine,
la 5,7-dichloro-6-(3-trifluorométhylphényl)-1,2,4-triazolo-[1,5-a]pyrimidine,
la 5,7-dichloro-6-(4-isopropylphényl)-1,2,4-triazolo-[1,5-a]pyrimidine,
la 5,7-dichloro-6-(4-trifluorométhoxyphényl)-1,2,4-triazolo-[1,5-a]pyrimidine,
la 5,7-dichloro-6-napht-2-yl-1,2,4-triazolo-[1,5-a]-pyrimidine,
la 5,7-dichloro-6-(3,4-diméthoxyphényl)-1,2,4-triazolo-[1,5-a]pyrimidine,
la 5,7-dichloro-6-(2-chlorophényl)-1,2,4-triazolo-[1,5-a]pyrimidine,
la 5,7-dichloro-6-(4-fluorophényl)-1,2,4-triazolo-[1,5-a]pyrimidine,
la 5,7-dichloro-6-(4-biphénylyl)-1,2,4-triazolo-[1,5-a]pyrimidine,
la 5,7-dichloro-6-(2-bromophényl)-1,2,4-triazolo-[1,5-a]pyrimidine,
la 5,7-dichloro-6-(2-fluorophényl)-1,2,4-triazolo-[1,5-a]pyrimidine,
la 5,7-dichloro-6-(4-phénoxyphényl)-1,2,4-triazolo-[1,5-a]pyrimidine,
la 5,7-dichloro-6-(4-benzyloxyphényl)-1,2,4-triazolo-[1,5-a]pyrimidine,
la 5,7-dichloro-6-(4-bromophényl)-1,2,4-triazolo-[1,5-a]pyrimidine,
la 5,7-dichloro-6-(3-bromophényl)-1,2,4-triazolo-[1,5-a]pyrimidine,
la 5,7-dichloro-6-(3-fluorophényl)-1,2,4-triazolo-[1,5-a]pyrimidine,
la 5,7-dichloro-6-(2-benzyloxyphényl)-1,2,4-triazolo-[1,5-a]pyrimidine,
la 5,7-dichloro-6-(2,3-diméthoxyphényl)-1,2,4-triazolo-[1,5-a]pyrimidine,
la 5,7-dichloro-6-(3,4,5-triméthoxyphényl)-1,2,4-triazolo-[1,5-a]pyrimidine, et
la 5,7-dichloro-6-(2-chloro-6-fluorophényl)-1,2,4-triazolo-[1,5-a]pyrimidine.

6. Procédé de préparation d'un composé de formule II telle que définie dans l'une quelconque des revendications précédentes, selon lequel on fait réagir un composé de formule générale V : 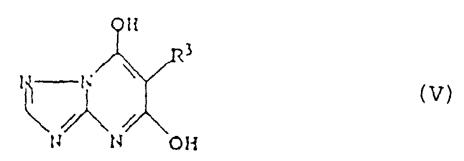 dans laquelle R³ est tel que défini dans l'une quelconque des revendications précédentes, avec un agent de chloration ou de bromation.

7. Procédé selon la revendication 6, dans lequel l'agent de chloration ou de bromation est l'oxychlorure de phosphore ou l'oxybromure de phosphore.
